# EUROPEAN PATENT APPLICATION

(11) **EP 3 967 372 A1**
(43) Date of publication of application: **16.03.2022**
(21) Application number: 20801924.0
(22) Date of filing: 01.05.2020
(51) Int. Cl.: A61Q 19/00, A61K 8/02, A61K 8/67, A61K 8/73, A61K 8/81

(54) **GEL-TYPE EXTERNAL COMPOSITION FOR SKIN**

(30) Priority: 07.05.2019 JP 2019087909
(71) Applicant: Hayashibara Co., Ltd., Okayama-shi, Okayama 702-8006 (JP)
(72) Inventor: AIZAWA Kiyomi, Okayama-shi Okayama 702-8006 (JP); MIYAKE Masaki, Okayama-shi Okayama 702-8006 (JP); MITSUZUMI Hitoshi, Okayama-shi Okayama 702-8006 (JP)
(74) Representative: Daniels, Jeffrey Nicholas
(86) International application number: PCT/JP2020/018452
(87) International publication number: WO 2020/226147

(57) **Abstract**

The present invention addresses the problem of providing a gel-type skin external composition containing a L-ascorbic acid derivative, which has excellent shape retention, stability, and excellent feeling of use even when salts coexist in the composition. The above problem is solved by providing a gel-type skin external composition containing a L-ascorbic acid derivative, a nonionic synthetic water-soluble polymer, and a natural water-soluble polysaccharide.

## Description

### Technical Field

The present invention relates to a gel-type skin external composition, and more particularly, to a gel-type skin external composition containing L-ascorbic acid derivatives with excellent shape retention, feeling of use, and stability.

### Background Art

It is known that L-ascorbic acid and L-ascorbic acid derivatives exhibit a wide range of effects such as a whitening effect, a collagen production promoting effect, and an antioxidant effect on the skin. Among these effects, for the purpose of whitening effect, a skin external composition containing a L-ascorbic acid derivative has been put into practical use for a long time and has been widely used by both young and elderly people.

Among these, gel-type skin external compositions have a property of having high water retention and retaining a large amount of moisture, and being able to blend moisture and oil in a well-balanced manner. Therefore, it is possible to impart a moisturizing effect which is strongly required to the skin external compositions, and it is also possible to formulate an aqueous or oily cosmetic component or the like, relatively easily. For these reasons, many gel-type skin external compositions have been put into practical use, such as gel-type lotions, gel-type emulsions, gel-type toners, gel-type creams, gel-type packs, gel-type sunscreens, gel-type cleansings, and gel-type foundations.

In the gel-type skin external compositions commonly used in the field of cosmetics and pharmaceuticals, thickeners are used in order to enhance its feeling of use, stability, and the like. For example, water-soluble synthetic carboxyvinyl polymers are commonly used for thickening of base agents of skin care products, because they have high thickening properties, form gels, and when applied to the skin, they easily break down while keeping moisture, and providing a non-sticky feel. On the other hand, water-soluble synthetic carboxyvinyl polymers have a disadvantage that they have low salt resistance, and when salts are added, the viscosity decreases significantly and the gel collapses.

If the concentration of the carboxyvinyl polymer is increased to compensate for the decrease in viscosity of the carboxyvinyl polymer due to salts, stickiness and rubbing dregs (scums derived polymer compounds) will occur, and the feeling of use will be significantly impaired. Therefore, in a gel-type skin external composition containing L-ascorbic acid derivatives, it has been difficult to design formulations containing carboxyvinyl polymers for the purpose of retaining a gel form, for example, in the case where salts are produced when neutralizing ascorbic acid derivatives, or when it is desired to formulate ascorbic acid derivatives in salt form.

On the other hand, in gel-type skin external compositions containing ascorbic acid derivatives, it has been proposed to use an associative thickener in which a hydrophobic group is introduced into the terminal of a nonionic water-soluble polymer chain to avoid the influence of salt on shape retention (Patent Document 1). This associative thickener is a nonionic hydrophobically modified polyurethane, and unlike carboxyvinyl polymers, it thickens through hydrophobic interactions between and within molecules, so that it is hardly affected by salts, and it is said that it is possible to formulate salts at a high concentration. On the other hand, gel-type skin external compositions containing a non-ionic hydrophobically modified polyurethane have an inconvenience that they do not easily stick to the fingers even if they were taken out with fingers from fully filled containers. In addition, the gel-type skin external compositions containing a non-ionic hydrophobically modified polyurethane were also not satisfactory in terms of feeling of use, such as spreadability on the skin and freshness.

In addition, it has also been proposed to use natural water-soluble polysaccharides as thickeners in a gel-type skin external composition containing ascorbic acid derivatives (Patent Document 2). By formulating natural water-soluble polysaccharides, the products become viscous solution, however, it was difficult to form gel formation. Further, the texture of the products was thick, not fresh, so the feeling of use was not sufficiently satisfactory.

In order to avoid the effect of salts on shape retention in gel-type skin external compositions containing ascorbic acid derivatives, the use of alkylmodified carboxyvinyl polymer (Patent Document 3), the use of a combination of lysophospholipid and glycerol monoalkylether (Patent Document 4), the use of polyacrylic acid derivatives (Patent Document 5), and the use of cross-linked hyaluronic acid gel (Patent Document 6) have also been proposed, but they were not sufficiently satisfactory in terms of shape retention and feeling of use. In gel-type topical skin compositions containing ascorbic acid derivatives, there is still a need for a thickening basal agent with excellent shape retention and feeling of use.

Furthermore, in recent years, there is a trend that skin external compositions having weakly acidic to neutral pH, which is close to pH of the skin, are favored. In the alkaline region of pH 7.5 or higher, there are concerns for example, about discoloration and decomposition of preservatives such as parabens, which are essential for aqueous formulations, and about odor change of moisturizing ingredients and the like. There are also many salt-type agents, such as ascorbic acid derivatives, whose stability decrease in the alkaline region. Therefore, for gel-type skin external compositions, it is desirable to have a thickening agent that is stable in the pH range of 5.0 to 7.0 and exhibits high shape retention and excellent feeling of use.

As described above, L-ascorbic acid derivatives have a whitening action and the like, and although it is a very useful component, gel-type skin external compositions containing the same have had problems in terms of shape retention, feeling of use such as low freshness, poor spreadability on the skin, hard to scoop with fingers, and generating rubbing dregs upon use, or the like. These problems have not been solved, and a gel-type skin external composition containing L-ascorbic acid derivatives having a sufficiently satisfactory shape retention, feeling of use, and stability has not yet been obtained.

### Prior Art Document

### Patent Document

[Patent Document 1] JP 2005-68023 A
[Patent Document 2] JP 2018-131471 A
[Patent Document 3] JP 2005-132828 A
[Patent Document 4] JP 2010-202605 A
[Patent Document 5] JP 2004-075672 A
[Patent Document 6] JP 2010-202522 A

### Disclosure of Invention

### Object of the Invention

The present invention has been made in view of the above-mentioned prior arts, and the problem to be solved is to provide a gel-type skin external composition containing L-ascorbic acid derivatives and having satisfactory shape retention, feeling of use, and stability, and in particular, to provide a gel-type skin external composition having a satisfactory shape retention, feeling of use, and stability without being affected by salts, even when it is necessary to contain L-ascorbic acid derivatives and salts thereof as a gel-type skin external composition, or when it is expected to generate salts by neutralization in order to adjust pH of the gel-type skin external composition from weakly acidic to near neutral.

### Means to Attain the Object

In order to solve the above problem, the present inventors conducted extensive studies. However, as a result of a preliminary test using various carboxyvinyl polymers which are commonly used as gel bases for skin external compositions, ionic synthetic water-soluble polymers such as various acrylic acidmethacrylate copolymers, and combination of these polymers with other materials, the problem of impaired shape retention of a composition containing L-ascorbic acid derivatives was not solved. Thus, it has been concluded that it is difficult to solve the above-mentioned problem by the combination of ionic synthetic water-soluble polymers and L-ascorbic acid derivatives.

The present inventors have compared and examined combinations of L-ascorbic acid derivatives and nonionic water-soluble polymers such as a PEG-240/HDI copolymer bis-decyltetradeceth-20 ether, and have found that, although the problem of impaired shape retention was eliminated or significantly reduced, other problems in terms of feeling of use such as poor spreadability on the skin, being hard to scoop with fingers, and rubbing dregs appeared.

Then, the present inventors broadly compared and examined the possibility of whether a problem of feeling of use such as poor spreadability on the skin, being hard to scoop with fingers, and rubbing dregs can be eliminated by combining other ingredients with compositions of L-ascorbic acid derivatives and nonionic synthetic water-soluble polymers such as PEG-240/HDI copolymer bis-decyltetradeceth-20 ether.

As a result, it has been found quite unexpectedly that, by further combining natural water-soluble polysaccharides or derivatives thereof, a gel-type skin external composition that meets the purposes was obtained, in which the problem of feeling of use such as poor spreadability on the skin, being hard to scoop with fingers, and rubbing dregs was eliminated or reduced, while showing excellent stability at the same time.

In other words, the present invention solves the above problems by providing a gel-type skin external composition containing a L-ascorbic acid derivative, a nonionic synthetic water-soluble polymer, and a natural water-soluble polysaccharide.

### Effects of Invention

The gel-type skin external composition of the present invention is a gel-type skin external composition comprising a L-ascorbic acid derivative, a nonionic synthetic water-soluble polymer, and a natural water-soluble polysaccharide, wherein the composition can be continuously and easily applied to the skin without discomfort by humans on a daily basis, and can be provided industrially and inexpensively. Even when a L-ascorbic acid derivative exhibiting various physiological functions is contained in the gel-type skin external composition of the present invention as described above, it is useful as a gel-type skin external composition which appropriately retains its physiological function, further has excellent shape retention and stability of formulation, and has remarkably excellent feeling of use.

### Mode for Carrying Out the Invention

Hereinafter, embodiments of the present invention are described, but they are merely illustrative of preferred embodiments in carrying out the present invention, and the present invention is not limited to these embodiments in any way.

The present invention relates to a gel-type skin external composition containing a L-ascorbic acid derivative, a nonionic synthetic water-soluble polymer, and a natural water-soluble polysaccharide.

The gel-type skin external composition of the present invention has an excellent feeling of use and its shape retention and stability are hardly affected even if it contains L-ascorbic acid derivatives and salts generated by neutralization of acids and bases in the composition.

The L-ascorbic acid derivatives referred to herein are not particularly limited as long as the derivatives themselves are more stable than L-ascorbic acid and liberate L-ascorbic acid in vivo so that the original function of L-ascorbic acid is exhibited. Examples of the derivatives of L-ascorbic acid which can be used as a component of the present invention include glycosyl derivatives, acylated derivatives, and phosphorylated derivatives of L-ascorbic acid.

As a glycosyl derivative of L-ascorbic acid, a derivative in which a glycosyl group is bonded to a hydroxyl group at 2-position of an L-ascorbic acid is preferably used because of high stability of the derivative. Examples of such derivatives include 2-O-α-glycosyl-L-ascorbic acid (ascorbic acid 2-glycoside) in which a series of glycosyl groups such as glucosyl groups, maltosyl groups, and maltotriosyl groups are α-bonded to a hydroxyl group at the 2-position of an L-ascorbic acid, as disclosed in, for example, JP 1991-135992 A and JP 1991-139288 A, and 2-O-β-D-galactosyl-L-ascorbic acid (ascorbic acid 2-galactoside) in which a galactosyl group is β-bonded to a hydroxyl group at the 2-position of an L-ascorbic acid, as disclosed in, for example, JP 1994-263790 A.

Further, examples of an acylated derivative (a fatty acid ester derivative) of L-ascorbic acid include ascorbic acid-palmitic acid ester, ascorbic acid-dipalmitic acid ester, ascorbic acid-tetrahexyldecanoic acid ester, and ascorbic acid-cholesterol ester, as disclosed in JP 1984-10505 A, JP 1988-104971 A, JP 1994-247956 A, JP 2004-331524 A, and the like. Further, examples of a phosphorylated derivative of L-ascorbic acid include ascorbic acid 2-phosphate ester and various metal salts thereof, as disclosed in JP 1986-152613 A, JP 1993-339123 A, JP 2002-3330 A, JP 2006-63060 A, and the like.

Among various derivatives of L-ascorbic acid described above, glycosyl derivatives of L-ascorbic acid are most preferably used due to their high stability, and among them, 2-O-α-D-glucosyl-L-ascorbic acid (hereinafter, abbreviated as "ascorbic acid 2-glucoside" in this specification) in which a glucosyl group is α-bonded to a hydroxyl group at the 2-position of L-ascorbic acid is the most suitable. Ascorbic acid 2-glucoside is a safe and highly versatile substance which has been widely used as a material for various foods and drinks, cosmetics, quasi-drugs, pharmaceuticals, and the like. In addition, ascorbic acid 2-glucoside is a derivative of L-ascorbic acid developed to solve the instability of L-ascorbic acid, and has revolutionary characteristics of not showing direct reducing properties, being stable, being excellent in preservation, and being able to be decomposed into L-ascorbic acid and D-glucose under the action of biological enzymes in living body to exert the original physiological activity of L-ascorbic acid. As disclosed in JP 1991-135992 A, JP 1991-139288 A, and the like, for example, ascorbic acid 2-glucoside is inexpensively produced on an industrial scale by a method in which cyclomaltodextrin glucanotransferase is applied as a glycosyltransferase to the solution containing L-ascorbic acid and starchy material to obtain ascorbic acid 2-glyciside, followed by glucoamylase treatment to the thus obtained ascorbic acid 2-glycoside.

The ascorbic acid 2-glucoside that is partly or completely in the form of crystals is suitable for use. An anhydrous crystal form of ascorbic acid 2-glucoside is known to exist and can be suitably used in the present invention. As a crystal of ascorbic acid 2-glucoside, a particulate composition containing anhydrous crystalline ascorbic acid 2-glucoside disclosed in the above-mentioned JP 1991-135992 A and International Publication WO2012/121297 pamphlet exists. As a commercially available product, there is a particulate composition containing anhydrous crystalline ascorbic acid 2-glucoside (product name "AA2G (registered trademark)", sold by Hayashibara Co., Ltd., Okayama, Japan).

The L-ascorbic acid derivative used in the gel-type skin external composition of the present invention may be in the form of a salt as long as it dissociates in an aqueous medium. Salts that are suitably used in the gel-type skin external composition of the present invention include sodium, potassium, aluminum, and zinc salts of ascorbic acid 2-glucoside. For example, the sodium salt of ascorbic acid 2-glucoside can be obtained by neutralizing an aqueous solution of ascorbic acid 2-glucoside with an alkali such as sodium hydroxide, and then drying. The sodium salt of ascorbic acid 2-glucoside is particularly suitable, like ascorbic acid 2-glucoside, for use in the present invention because when dissolved in an aqueous medium, it dissociates into ascorbic acid 2-glucoside anions and sodium ions at least in part depending on the pH.

The term "a non-ionic synthetic water-soluble polymer" as used in this specification refers to a non-ionic water-soluble polymer obtained by chemical synthesis from petroleum or the like as raw materials. Suitable examples include polyvinyl alcohol, polyvinyl pyrrolidone, polyvinyl methyl ether, PEG-240/HDI copolymer bis-decyltetradeceth-20 ether, polyurethane, polyethylene oxide, ethylene oxide, and polyvinyl ether, polyethylene oxide, ethylene oxide-propylene oxide block copolymers, polymer compound silicones, and derivatives thereof.

Among these, PEG-240/HDI copolymer bis-decyltetradeceth-20 ether, polyurethane, polyvinyl alcohol, ethylene oxide-propylene oxide block copolymer and their derivatives can be used more suitably.

Natural water-soluble polysaccharides, as used herein, may be natural products and/or polysaccharides made from natural products among water-soluble polymers, and may further include the ones obtained by chemically and enzymatically modifying them. The said natural products may be of any origin, for example, plants, microbials, animals, or seaweed. Suitable examples include guar gums such as guar gum, cationized guar gum, carboxymethylhydroxypropylated guar gum, and hydroxypropylated guar gum; soluble celluloses such as quince seed gum, mannan, tamarind gum, cod gum, soluble dextrin, locust bean gum, gum arabic, ghatti gum, karaya gum, tragacanth gum, arabinogalactan, pectin, marmello, cationized cellulose, methylcellulose, ethyl cellulose, hydroxymethylcellulose, hydroxypropyl cellulose, hydroxypropylmethylcellulose, and carboxypropylmethylcellulose; plant-derived polymers such as soluble starch, carboxymethyl starch, methyl starch, starch phosphate, sodium starch glycolate, and their derivatives and their salts; animalderived polymers such as chitosan, hyaluronic acid, chondroitin sulfate, and their derivatives and their salts; microbial-derived polymers such as cardran, xanthan gum, gellan gum, cyclodextrin, dextran, pullulan, succinoglucan, and their derivatives and their salts; and seaweed-derived polymers such as alginic acid, carrageenan, agar, agarose, furcellan, propylene glycol alginate, and their derivatives and their salts.

Among these, soluble cellulose, xanthan gum, guar gum, tamarind gum, locust bean gum, soluble starch, soluble dextrin, cyclodextrin, pullulan, their derivatives and their salts can be used more suitably.

The mass ratio of the L-ascorbic acid derivative to the nonionic synthetic water-soluble polymer and the natural water-soluble polysaccharide contained in the gel-type skin external composition of the present invention on a dry solid basis is preferably in the range of 1 : 0.01 to 500 : 0.01 to 500, more preferably in the range of 1 : 0.05 to 200 : 0.02 to 100, and more further preferably in the range of 1 : 0.1 to 100 : 0.05 to 50. If the mass ratio is below the lower limit of the above-mentioned formulating ratio, or if the mass ratio is above the upper limit of the above-mentioned formulating ratio, the desired effect may be significantly reduced or may not be exhibited. Thus, such ratio is not desirable. When nonionic synthetic water-soluble polymers contained in the gel-type skin external composition of the present invention are formulated above this range, the gel-type skin external composition is inferior in feeling of use, for example generation of rubbing dregs or the like, and when the composition is formulated below this range, the gel-type skin external composition is not desirable because it is inferior in shape retention property. When natural water-soluble polysaccharides contained in the gel-type skin external composition of the present invention are usually formulated above this range, the gel-type skin external composition is inferior in feeling of use, for example generation of stickiness or the like, and when the composition is formulated below this range, the gel-type skin external composition is not desirable because the feeling of use improving effect is hardly obtained and the feeling of use is inferior.

Although the gel-type skin external composition of the present invention can be used alone as a gel-type skin external composition, it is preferable to use the gel-type skin external composition as various cosmetics by adding appropriate cosmetic ingredients that are generally used depending on the purpose of use. For example, the gel-type skin external composition of the present invention may be used as a gel-type lotion by adding ethanol or a moisturizer to the composition, and such gel-type lotion may be further formulated with one or more astringents, keratin softeners, emollients, or surfactants. In addition, it is also arbitrarily incorporated with one or more fragrances, dyes, preservatives or disinfectants, UV absorbers or UV scatterers, metal sequestering agents, buffers (pH adjusters), antioxidants, whitening agents, antiinflammatory agents, blood circulation promoters, thickeners, vitamins, amino acids, cell activators, transdermal absorption promoters, solubilizers, pigments, and other ingredients and the like, and those which can be generally used in skin external compositions including cosmetics can be used, and in addition to the above ingredients, any existing cosmetic materials can be further used; For example, all the cosmetic materials described in the following publications can be appropriately used in combination: "Supplements to The Japanese Standards of Cosmetic Ingredients", 2nd Edition, edited by Japan Cosmetic Industry Association, published by Yakuji Nippo, Ltd., Tokyo, Japan, in 1984; "The Japanese Cosmetic Ingredients Codex", supervised by Evaluation and Registration Division, Pharmaceutical Affairs Bureau, Ministry of Health and Welfare, published by Yakuji Nippo, Ltd., Tokyo, Japan, in 1993; "Supplement to The Japanese Cosmetic Ingredients Codex" supervised by Evaluation and Registration Division, Pharmaceutical Affairs Bureau, Ministry of Health and Welfare, published by Yakuji Nippo, Ltd., Tokyo, Japan, in 1993; "The Comprehensive Licensing Standards of Cosmetics by Category", supervised by Evaluation and Registration Division, Pharmaceutical Affairs Bureau, Ministry of Health and Welfare, published by Yakuji Nippo, Ltd., Tokyo, Japan, in 1993; "Japanese Pharmaceutical Codex (JPC)", supervised by Evaluation and Registration Division, Pharmaceutical Affairs Bureau, Ministry of Health and Welfare, published by Yakuji Nippo, Ltd., Tokyo, Japan, in 1997; "The Latest Cosmetic Science (revised and enlarged II)", edited by The Society of Cosmetic Chemists of Japan, published by Yakuji Nippo, Ltd., Tokyo, Japan, in 1992, "Encyclopedia of Cosmetics", edited by The Society of Cosmetic Chemists of Japan, published by Maruzen Co., Ltd., Tokyo, Japan in 2003; and "Japan Cosmetic Raw Material Collection 2007", edited by Japan Cosmetic Industry Association, published by Yakuji Nippo, Ltd., Tokyo, Japan, in 2007.

The amount of the above-mentioned general-purpose cosmetic ingredients to be formulated in the gel-type skin external composition of the present invention is optional as long as the desired effect is achieved. As a method of formulating, one or more known methods of inclusion, such as admixing, kneading, mixing, adding, dissolving, dipping, penetrating, spreading, coating, spraying, and injecting, can be used appropriately in the processes until the production of the composition is completed.

The gel-type skin external composition of the present invention can be suitably used in the field of cosmetics such as skin care cosmetics, make-up cosmetics, and hair care cosmetics. In particular, their excellent effects can be expected when they are formulated into skin care cosmetics such as lotions, facial washes, serums, emulsions, and creams that are applied directly to the skin. It is also optional that the gel-type skin external composition of the present invention is suitably formulated and utilized in other cosmetics, quasi-drugs, or pharmaceuticals.

The present invention is explained in more detail by the following experiments.

### <Experiment 1: Preparation of gel-type skin external composition containing L-ascorbic acid derivatives and evaluation of their properties and feeling of use>

Tests for shape retention and feeling of use were performed on gel-type skin external compositions containing an L-ascorbic acid derivative.

### <Experiment 1-1: Preparation of a gel-type skin external composition containing an L-ascorbic acid derivative>

An ascorbic acid derivative-containing gel-type skin external composition was prepared according to the formulation shown in Table 1 using ascorbic acid 2-glucoside as an ascorbic acid derivative, PEG-240/HDI copolymer bis-decyltetradeceth-20 ether as a nonionic synthetic water-soluble polymer, and cellulose derivative as a natural water-soluble polysaccharide, respectively.

After putting 215 g of deionized water into a glass container, 20 g of PEG-240/HDI copolymer bis-decyltetradeceth-20 ether (product name "Adekanol GT-730", sold by ADEKA Corporation, Tokyo, Japan) and 15 g of pentylene glycol (product name "Hydrolite-5", sold by Symrise K.K., Tokyo, Japan) as a preservative were added, and mixed at 300 rpm using a three one motor (BL1200, sold by Yamato Scientific Co., Ltd., Tokyo, Japan) to prepare a nonionic synthetic water-soluble polymer aqueous solution (2.4% by mass on a dry solid basis of PEG-240/decyltetradeceth-20/HDI) copolymer (hereinafter, referred to as "nonionic synthetic water-soluble polymer aqueous solution"). "Adekanol GT-730" is a liquid composition consisting of 30% by mass of PEG-240/HDI copolymer bis-decyltetradeceth-20 ether, 50% by mass of butylene glycol, 19.94% by mass of water, 0.03% by mass of potassium laurate, and 0.03% by mass of tocopherol. Next, 225 g of deionized water heated to 70 °C was put into another glass container, and then 25 g of hydroxypropyl methylcellulose stearoxy ether (product name "Sangelose 60 L", sold by Daido Chemical Corporation, Osaka, Japan) was added, and stirred using a stirrer for 1 minute. Then a natural water-soluble polysaccharide aqueous solution (hydroxypropyl methylcellulose stearoxy ether concentration: 10.0% by mass) (hereinafter, referred to as "natural water-soluble polysaccharide aqueous solution") is prepared by stirring for 30 minutes while cooling the container with ice water. Further, 20 g of particulate composition containing anhydrous crystalline ascorbic acid 2-glucoside (product name "AA2G (registered trademark)", sold by Hayashibara Co., Ltd., Okayama, Japan) was dissolved using a stirrer after being added to 100 g of deionized water, followed by the addition of 20 g of citrate buffer solution (1% by mass of citric acid, 15% by mass of sodium citrate) and 24.7 g of 10% by mass sodium hydroxide for preparation of neutralized ascorbic acid 2-glucoside solution (total amount 164.7 g, (hereinafter referred to as "ascorbic acid 2-glucoside neutralized aqueous solution")). For each composition, 30 g of deionized water is placed in a separate glass container, and after adding a nonionic synthetic water-soluble polymer solution and a natural water-soluble polysaccharide solution to achieve the compositions shown in Table 1, 32.94 g of ascorbic acid 2-glucoside neutralized water solution is added. After that, while mixing using a spatula, an appropriate amount of 10 % by mass sodium hydroxide was added, and the pH was adjusted to around 6.0 using a pH meter (F-74, sold by HORIBA, Ltd., Kyoto, Japan) and then water was added to the total amount of 200 g, and thus skin external compositions 1 to 8 (hereinafter, simply referred to as "compositions 1 to 8") were obtained.

The formulation of compositions 1 to 8 is shown in Table 1. Specifically, for compositions 1 to 7, the amount of PEG-240/HDI copolymer bis-decyltetradeceth-20 ether was the same (1.2% by mass), and the amount of hydroxypropyl methylcellulose stearoxy ether varied to 0, 0.1, 0.2, 0.3, 0.5, 1, or 5 % by mass. For composition 8, PEG-240/HDI copolymer bis-decyltetradeceth-20 ether was not included in the formulation, but hydroxypropyl methylcellulose stearoxy ether was included at 5% by mass.

Controls 1 and 2 were prepared as follows according to the formulations shown in Table 1. Specifically, for controls 1 and 2, they were prepared in the same manner as in compositions 1 to 8, except that 0.5% by mass of carboxyvinyl polymer as an ionic synthetic water-soluble polymer or 0.5% by mass of acrylic acid-alkyl methacrylate copolymer as an ionic synthetic water-soluble polymer was formulated in place of PEG-240/HDI copolymer bis-decyltetradeceth-20 ether as a nonionic synthetic water-soluble polymer, and that hydroxypropyl methylcellulose stearoxy ether as a natural water-soluble polysaccharide was not included. The carboxyvinyl polymer and the acrylic acid-alkyl methacrylate copolymer were formulated by preparing "ionic synthetic water-soluble polymer aqueous solutions" containing a carboxyvinyl polymer or an acrylic acid-alkyl methacrylate copolymer in advance, and using these solutions instead of the above "nonionic synthetic water-soluble polymer aqueous solution". A method for preparing an "ionic synthetic water-soluble polymer aqueous solution" is as follows. After adding 215 g of deionized water heated to 45°C to a glass container, 2.5 g of carboxyvinyl polymer (product name "AQUPEC HV-505" (sold by Sumitomo Seika Chemicals Co., Ltd., Osaka, Japan) or acrylic acid-alkyl methacrylate copolymer (product name "Carbopol Ulyrez10 Polymer" (sold by Lubrizol Advanced Materials Inc., Avon Lake, United States), and 15 g of pentylene glycol as a preservative were added and mixed using a stirrer (level 4), and pH were adjusted to around 6.0 using a pH meter by adding an appropriate amount of 10% by mass sodium hydroxide, and then deionized water was added to make the total volume of 250 g to prepare an ionic synthetic water-soluble polymer aqueous solution (1.0% by mass of solid concentration).

Controls 3 and 4 were prepared in the same manner as compositions 1 to 8, except that 1% by mass of sorbitol or trehalose was added as an oligosaccharide in place of hydroxypropyl methylcellulose stearoxy ether as a natural water-soluble polysaccharide, based on the composition shown in Table 1. Specifically, 225 g of deionized water was placed in a glass container, and then 25 g of sorbitol (product name "Sorbitol Kao", sold by Kao Corporation, Tokyo, Japan) or 25 g of trehalose (product name "Trehalose (cosmetics grade)", sold by Hayashibara Co., Ltd., Okayama, Japan) was added and stirred using a stirrer for 1 minute to prepare an oligosaccharide solution, which was used in place of an aqueous natural water-soluble polysaccharide solution.

### <Experiment 1-2: Evaluation of the properties of gel-type skin external compositions containing an L-ascorbic acid derivative>

Compositions 1 to 8 and controls 1 to 4 prepared in Experiment 1-1 (hereinafter referred to as "each composition") were evaluated for properties and feeling of use, and the results were shown in Table 2.

The property of each composition was evaluated using shape retention as an index. One hundred and ten mL of each composition immediately after preparation was filled into a transparent glass bottle with a screw cap with 4 cm in diameter and 12 cm in height (No. 8, Maruemu Corporation, Osaka, Japan). After being left to stand for a day and night, each composition was then observed macroscopically that how the morphology of each composition changed when the bottle was tilted 45° from the vertical direction to the horizontal direction, namely shape retention was observed. In compositions having no salt resistance and without gel formation, the composition is unstable, and therefore the composition flows due to gravity changes when the bottle is tilted. This test/evaluation is an indicator of the aesthetic appearance of the gel-forming composition in the state of being filled in the cosmetic container. The properties of each composition were visually observed and judged in three levels based on the following criteria.

### (Judgment Criteria for Shape retention)

∘: When the bottle is tilted, the composition retains its shape, or is deformed but no fluidity is observed.
△: When the bottle is tilted, the composition is deformed and showed gentle fluidity.
x_{:} When the bottle is tilted, and if the cap is opened, the composition is fluid enough to flow down.

The feeling of use each of composition (freshness, spreadability on the skin and rubbing dregs) was evaluated by five trained panelists (two males and three females) using each composition prepared in the same method as in Experiment 1-1 and left to stand for a day and night. The measurement site was the inner forearm of the panelists. 0.5 g of each composition was placed on the inner side of the panelist's forearm and spread with the finger pad of the other hand, and sensory evaluation was performed on the freshness of the composition immediately after spreading and its spreadability on the skin. In addition, a finger was moved back and forth until each composition was dried, and a sensory evaluation was performed on the presence or absence of rubbing dregs. In addition, another finger was used for sensory evaluation of the amount of scooping with fingers by adhering each composition to the finger. The feeling of use was determined in three levels based on the following criteria.

### (Judgment Criteria for freshness)

∘ : Four or more panelists assessed as fresh.
△ : Two or three panelists assessed as fresh.
x : One or less panelist assessed as fresh.

### (Judgment Criteria for spreadability on the skin)

∘ : Four or more panelists assessed as having good spreadability.
△ : Two or three panelists assessed as having good spreadability.
x : One or less panelist assessed as having good spreadability.

### (Judgment Criteria for scoopability with fingers)

∘ : Four or more panelists assessed as scooping with fingers well.
△ : Two or three panelists assessed as scooping with fingers well.
x : One or less panelist assessed as scooping with fingers well.

### (Judgment Criteria for rubbing dregs)

∘ : One or less panelist assessed as generating rubbing dregs.
△ : Two or three panelists assessed as generating rubbing dregs.
× : Four or more panelists assessed as generating rubbing dregs.

The observed results of these properties and feeling of use of each composition are shown in Table 2 below.

**[Table 1]**

| | Control 1 | Control 2 | Control 3 | Control 4 | Compos ition 1 | Compos ition 2 | Compos ition 3 | Compos ition 4 | Compos ition 5 | Compos ition 6 | Compos ition 7 | Compos ition 8 |
|---|---|---|---|---|---|---|---|---|---|---|---|---|
| Ascorbic acid 2-glucoside | 2 | 2 | 2 | 2 | 2 | 2 | 2 | 2 | 2 | 2 | 2 | 2 |
| Carboxyvinyl polymer | 0.5 | - | - | - | - | - | - | - | - | - | - | - |
| Acrylic acid-alkyl methacrylate copolymer | - | 0.5 | - | - | - | - | - | - | - | - | - | - |
| PEG-240/HDI copolymer bis-decyltetradec eth-20 ether | - | - | 1.2^{*} | 1.2^{*} | 1.2^{*} | 1.2^{*} | 1.2^{*} | 1.2^{*} | 1.2^{*} | 1.2^{*} | 1.2^{*} | - |
| Sorbitol | - | - | 1 | - | - | - | - | - | - | - | - | - |
| Trehalose | - | - | - | 1 | - | - | - | - | - | - | - | - |
| Hydroxyprop yi | - | - | - | - | 0 | 0.1 | 0.2 | 0.3 | 0.5 | 1 | 5 | 5 |
| methylcellulo se stearoxy ether | | | | | | | | | | | | |
| Pentylene glycol | 1.5 | 1.5 | 1.5 | 1.5 | 1.5 | 1.5 | 1.5 | 1.5 | 1.5 | 1.5 | 1.5 | 1.5 |
| Citrate buffer | 2 | 2 | 2 | 2 | 2 | 2 | 2 | 2 | 2 | 2 | 2 | 2 |
| 10% by mass of sodium hydroxide aqueous solution | q.s. | q.s. | q.s. | q.s. | q.s. | q.s. | q.s. | q.s. | q.s. | q.s. | q.s. | q.s. |
| Deionized water | remai ning | remai ning | remai ning | remai ning | remainin 9 | remainin 9 | remainin 9 | remainin 9 | remainin 9 | remainin 9 | remainin 9 | remainin 9 |
| Total | 100 | 100 | 100 | 100 | 100 | 100 | 100 | 100 | 100 | 100 | 100 | 100 |

| | | | | | | | | | | | | |
|---|---|---|---|---|---|---|---|---|---|---|---|---|
| (unit: % by mass) ^{*} : As dry solid content of PEG-240/HDI copolymer bis-decyltetradeceth-20 | | | | | | | | | | | | |

**[Table 2]**

| | Cont rol1 | Cont rol2 | Cont rol3 | Cont rol4 | Compos ition 1 | Compos ition2 | Compos ition3 | Compos ition4 | Compos ition5 | Compos ition6 | Compos ition7 | Compos ition8 |
|---|---|---|---|---|---|---|---|---|---|---|---|---|
| Property/ Shape retention | × | × | ○ | ○ | ○ | ○ | ○ | ○ | ○ | ○ | ○ | × |
| Feeling of use/ Freshness | ○ | ○ | × | × | × | △ | ○ | ○ | ○ | ○ | △ | × |
| Feeling of use/ Spreadability on the skin | × | × | × | × | × | ○ | ○ | ○ | ○ | ○ | ○ | ○ |
| Feeling of use/ Scoopability with fingers | × | × | × | × | × | ○ | ○ | ○ | ○ | ○ | ○ | ○ |
| Feeling of use/ Rubbing dregs | × | × | × | × | × | ○ | ○ | ○ | ○ | ○ | ○ | ○ |

As shown in Table 2, where the shape retention of control 1 and control 2 were both evaluated as "x", when carboxyvinyl polymer or acrylic acid-alkyl methacrylate copolymer, as an ionic synthetic water-soluble polymer, was formulated in place of PEG-240/HDI copolymer bis-decyltetradeceth-20 ether as a nonionic synthetic water-soluble polymer, no gel was formed in both cases, that is, no shape retention of the gel was observed. On the other hand, as shown by the result that the shape retention of compositions 1 to 7 were all evaluated as "o", when PEG-240/HDI copolymer bis-decyltetradeceth-20 ether as a nonionic synthetic water-soluble polymer was formulated, a gel was formed in any of the compositions, that is, the shape retention of the gel was confirmed. Furthermore, in the case of composition 8, which was not blended with PEG-240/HDI copolymer bis-decyltetradeceth-20 ether, although it became a viscous solution, no gel was formed and the shape retention was evaluated as "x". From these results, it was confirmed that it is necessary to formulate PEG-240/HDI copolymer bis-decyltetradeceth-20 ether as a nonionic synthetic water-soluble polymer for the gel formation. This is also supported by the result that the shape retention of control 3 and control 4 including PEG-240/HDI copolymer bis-decyltetradeceth-20 ether as a nonionic synthetic water-soluble polymer, was also evaluated as "o".

However, the feeling of use of control 3 and control 4 formulated with sorbitol or trehalose in place of hydroxypropyl methylcellulose stearoxy ether as a natural water-soluble polysaccharide, and composition 1, which did not contain hydroxypropyl methylcellulose stearoxy ether as a natural water-soluble polysaccharide, were evaluated as "x" for "freshness", "spreadability on the skin", "scoopability with fingers", and "rubbing dregs", which were poor. On the other hand, the feeling of use of compositions 2 to 8, in which the amount of hydroxypropyl methylcellulose stearoxy ether was from 0.1 to 5% by mass, were evaluated as "∘" with respect to "spreadability on the skin", "scoopability with fingers", and "rubbing dregs". With respect to "freshness" in the feeling of use, composition 2 in which the amount of hydroxypropyl methylcellulose stearoxy ether was 0.1% by mass, and composition 7 in which the amount of hydroxypropyl methylcellulose stearoxy ether was 5% by mass were evaluated as "△", and even if the amount of hydroxypropyl methylcellulose stearoxy ether was 5% by mass, composition 8, which does not contain PEG-240/HDI copolymer bis-decyltetradeceth-20 ether, was evaluated as "x". From these results, it became clear that, with respect to the feeling of use, the formulating concentration of hydroxypropyl methylcellulose stearoxy ether as a natural water-soluble polysaccharide, is preferably in the range of 0.1 to 5% by mass, and particularly, with respect to "freshness", the range of 0.2 to 1% by mass is more preferable. In addition, it was confirmed that it is necessary to formulate natural water-soluble polysaccharides such as hydroxypropyl methylcellulose stearoxy ether instead of oligosaccharides such as sorbitol and trehalose to improve the feeling of use.

### <Experiment 2: Preparation of gel-type skin external composition containing L-ascorbic acid derivatives and evaluation of their properties and feeling of use>

The gel-type skin external composition containing L-ascorbic acid derivatives was tested for its shape retention and usability by varying the type of natural water-soluble polysaccharides.

### <Experiment 2-1 : Preparation of gel-type skin external compositions containing an L-ascorbic acid derivative>

A natural water-soluble polysaccharide aqueous solution was prepared in the same manner as in Experiment 1-1, except that as a natural water-soluble polysaccharide, xanthan gum (product name "Echogum T/Keltrol T", sold by DSP GOKYO Food & Chemical Co., Ltd., Osaka, Japan), guar gum (product name "SUPERGEL CSA 200/50", sold by Sansho Co., Ltd., Osaka, Japan) or locust bean gum (product name GENU GUM type RL-200-J, sold by Sansho Co., Ltd., Osaka, Japan) were used in place of hydroxypropyl methylcellulose stearoxy ether (product name "Sangelose 60L"), and compositions 9 to 16 were prepared. The composition of the formulation is shown in Table 3, and in detail, for compositions 9 to 13, the amount of the PEG-240/HDI copolymer bis-decyltetradeceth-20 ether was 1.2% by mass in the same amount, and the amount of the xanthan gum was varied to 0, 0.1, 0.2, 0.5, or 1% by mass. For the composition 14, the PEG-240/HDI copolymer bis-decyltetradeceth-20 ether was not contained, and xanthan gum was contained at 1% by mass. For the compositions 15 and 16, the amount of the PEG-240/HDI copolymer bis-decyltetradeceth-20 ether was same in both compositions at 1.2% by mass, and guar gum or locust bean gum was formulated instead of xanthan gum, and the amount was 0.2% by mass.

### <Experiment 2-2: Evaluation of the properties and usability of gel-type skin external compositions containing an L-ascorbic acid derivative>

The evaluation of the properties/the shape retention and the feeling of use of compositions 9 to 16 obtained in Experiment 2-1 was performed in the same manner as in Experiment 1-2, and the results are shown in Table 4. In addition, the viscosity of the compositions was measured to evaluate their properties and is also shown in Table 4. The viscosity of each composition was measured 3 times for each sample using a rheometer (MCP102, sold by Anton Paar Gmbh., Graz, Austria) and a measuring jig CP-50-1-SN34132 under the conditions of a shear rate of 101/sec, a data point of 10, and a measurement interval of 6 seconds, and the average value thereof was obtained.

**[Table 3]**

| | Compositio n 9 | Compositio n 10 | Compositio n 11 | Compositio n 12 | Compositio n 13 | Compositio n 14 | Compositio n 15 | Compositio n 16 |
|---|---|---|---|---|---|---|---|---|
| Ascorbic acid 2-glucoside | 2 | 2 | 2 | 2 | 2 | 2 | 2 | 2 |
| PEG-240/ HDI copolymer bis-decyltetradeceth-20 ether | 1.2^{*} | 1.2^{*} | 1.2^{*} | 1.2^{*} | 1.2^{*} | 0 | 1.2^{*} | 1.2^{*} |
| Xanthan gum | 0 | 0.1 | 0.2 | 0.5 | 1 | 1 | - | - |
| Guar gum | - | - | - | - | - | - | 0.2 | - |
| Locust bean gum | - | - | - | - | - | - | - | 0.2 |
| Pentylene glycol | 1.5 | 1.5 | 1.5 | 1.5 | 1.5 | 1.5 | 1.5 | 1.5 |
| Citrate buffer | 2 | 2 | 2 | 2 | 2 | 2 | 2 | 2 |
| 10% by mass of sodium hydroxide aqueous solution | q.s. | q.s. | q.s. | q.s. | q.s. | q.s. | q.s. | q.s. |
| Deionized water | remaining | remaining | remaining | remaining | remaining | remaining | remaining | remaining |
| Total | 100 | 100 | 100 | 100 | 100 | 100 | 100 | 100 |

| | | | | | | | | |
|---|---|---|---|---|---|---|---|---|
| (unit: % by mass) ^{*} : As dry solid content of PEG-240/HDI copolymer bis-decyltetradeceth-20 | | | | | | | | |

**[Table 4]**

| | Composition9 | Composition10 | Composition11 | Composition12 | Composition13 | Composition14 | Composition15 | Composition16 |
|---|---|---|---|---|---|---|---|---|
| Property/Viscosity (mPa·s) | 4450 | 4824 | 4883 | 4729 | 4884 | no data | 4133 | 4064 |
| Property/Shape retention | ○ | ○ | ○ | ○ | ○ | x | ○ | ○ |
| Feeling of use/Freshness | x | △ | ○ | ○ | ○ | x | ○ | ○ |
| Feeling of use/Spreadability on the skin | x | ○ | ○ | ○ | ○ | ○ | ○ | ○ |
| Feeling of use/Scoopability with fingers | x | ○ | ○ | ○ | ○ | ○ | ○ | ○ |
| Feeling of use/Rubbing dregs | x | ○ | ○ | ○ | ○ | ○ | ○ | ○ |

As shown in the test results of Table 4, for compositions 9 to 13 containing 1.2% by mass of PEG-240/HDI copolymer bis-decyltetradeceth-20 ether, the "property/viscosity" that numerically expresses the shape retention property, showed almost the same value regardless of the presence or absence of xanthan gum and the amount thereof. In addition to this, the "property/shape retention" of each of the compositions 9 to 13 was evaluated as "o", that is, the gel was formed and retained its shape. On the other hand, as shown in the results of composition 14, without PEG-240/HDI copolymer bis-decyltetradeceth-20 ether, although a viscous solution was obtained, no gel was formed and "property/shape retention" was evaluated as "x". In other words, it was confirmed that a gel could be formed by formulating PEG-240/HDI copolymer bis-decyltetradeceth-20 ether as a nonionic synthetic water-soluble polymer. In addition, with respect to the feeling of use, as shown in Table 4, the feeling of use of the composition 9 without xanthan gum was evaluated as "x" in any of "freshness", "spreadability on the skin", "scoopability with fingers", and "rubbing dregs", which was poor. In contrast, in the case of compositions 11 to 13 containing xanthan gum in a range of 0.2 to 1 % by mass, the feeling of use thereof was evaluated as "∘" in all of "freshness", "spreadability on the skin", "scoopability with fingers", and "rubbing dregs. On the other hand, in the case of the composition 10, which contains relatively small amount of xanthan gum of 0.1% by mass, it was evaluated as "∘" for 3 items of "spreadability on the skin", "scoopability with fingers", and "rubbing dregs", but was evaluated as "△" for "freshness", indicating slightly inferior in the feeling of use. Further, the "feeling of use (freshness)" of the composition 14 containing 1% by mass of xanthan gum but not containing PEG-240/HDI copolymer bis-decyltetradeceth-20 ether was evaluated as "x", also indicating the slightly inferior feeling of use. In addition, in the case of the compositions 15 and 16 containing 0.2% by mass of guar gum or locust bean gum in place of xanthan gum, as in the case of containing xanthan gum, the "viscosity" thereof showed almost the same value as in the case of formulating xanthan gum, as its property. In addition, the "shape retention" thereof was evaluated as "∘", and the feeling of use were evaluated as "∘" in any of "freshness", "spreadability on the skin", "scoopability with fingers" and "rubbing dregs". The above results indicate that even when xanthan gum is used as a natural water-soluble polysaccharide, as in the case of hydroxypropyl methylcellulose stearoxy ether, it is preferable to use xanthan gum in the range of 0.1 to 1% by mass, and more preferable in the range of 0.2 to 1% by mass to obtain a better feeling of use. It also shows that guar gum and locust bean gum can be preferably used as natural water-soluble polysaccharides.

The results in Table 2 and Table 4 show that the skin external compositions containing ascorbic acid 2-glucoside can form a gel when PEG-240/HDI copolymer bis-decyltetradeceth-20 ether, a non-ionic synthetic water-soluble polymer, is added, but the feeling of use is poor. On the other hand, in combination with these polysaccharides, the use of natural water-soluble polysaccharides such as hydroxypropyl methylcellulose stearoxy ether, or xanthan gum, guar gum, or locust bean gum has been shown to significantly improve the feeling of use.

### <Experiment 3: Storage stability test of gel-type skin external compositions containing an L-ascorbic acid derivative>

Storage stability test was performed on the gel-type skin external compositions containing an L-ascorbic acid derivative.

### <Experiment 3-1: Preparation of gel-type skin external compositions containing an L-ascorbic acid derivative for storage stability test>

In order to test the storage stability, gel-type skin external compositions 17 and 18 containing an L-ascorbic acid derivative (hereinafter, simply referred to as "compositions 17 and 18") were prepared in the same manner as in Experiment 1-1, except that 10% by mass of sodium hydroxide was substituted for 10% by mass of potassium hydroxide. The composition of the formulations is shown in Table 5. For compositions 17 and 18, the amount of PEG-240/HDI copolymer bis-decyltetradeceth-20 ether was both 1.2% by mass, and the amount of hydroxypropyl methylcellulose stearoxy ether was 0.2 or 0.3% by mass. Controls 5 and 6 were prepared in the same manner as compositions 17 and 18, except that they did not contain ascorbic acid 2-glucoside and 10% by mass of potassium hydroxide. 110 mL of each composition and each control immediately after preparation was filled into a transparent glass bottle with a screw cap having a diameter of 4 cm and a height of 12 cm, sealed, and stored in an incubator (CSH-111, manufactured by ESPEC Corp., Osaka, Japan) set at 40 °C for 3 months. The stability of each composition and each control after storage was evaluated over time immediately after preparation, after 1 month of storage, and after 3 months of storage.

### <Experiment 3-2: Evaluation of properties and feeling of use of gel-type skin composition containing an L-ascorbic acid derivative for storage stability test>

The evaluation of properties and feeling of use of each composition and controls 5 and 6, after storage at 40 °C for a predetermined period of time, was performed as an assessment of stabilities thereof. As evaluation items of properties, viscosity, pH, coloration, and shape retention were evaluated. Viscosity and shape retention were evaluated in the same manner as in Experiment 2-2. The pH was measured using a pH meter. The coloration was visually observed. In addition, as an evaluation item of feeling of use, freshness, spreadability on the skin, scoopability with fingers, and rubbing dregs were evaluated in the same manner as in Experiment 1-2. The results obtained are summarized in Table 6.

**[Table 5]**

| | Control 5 | Control 6 | Composition 17 | Conposition 18 |
|---|---|---|---|---|
| Ascorbic acid 2-glucoside | 0 | 0 | 2 | 2 |
| PEG-240/HDI copolymer bis-decyltetradeceth-20 ether | 1.2^{*} | 1.2^{*} | 1.2^{*} | 1.2^{*} |
| Hydroxypropyl methylcellulose stearoxy ether | 0.2 | 0.3 | 0.2 | 0.3 |
| Pentylene glycol | 1.5 | 1.5 | 1.5 | 1.5 |
| Citrate buffer | 2 | 2 | 2 | 2 |
| 10% by mass of potassium hydroxide aqueous solution | 0 | 0 | q.s. | q.s. |
| Deionized water | remaining | remaining | remaining | remaining |
| Total | 100 | 100 | 100 | 100 |

| | | | | |
|---|---|---|---|---|
| (unit: % by mass) ^{*} : As dry solid content of PEG-240/HDI copolymer bis-decyltetradeceth-20 | | | | |

**[Table 6]**

| | | Control 5 | Control 6 | Composition 17 | Composition 18 |
|---|---|---|---|---|---|
| Property/Viscosity (mPa·s) | immediately after preparation | 6727 | 9260 | 6665 | 8657 |
| | After 1 month storage at 40°C | 6600 | 8309 | 6395 | 7757 |
| | After 3 months storage at 40°C | 7298 | 8154 | 7121 | 9032 |
| Property/pH | immediately after preparation | 6.5 | 6.5 | 6.3 | 6.4 |
| | After 1 month storage at 40°C | 6.3 | 6.4 | 6.2 | 6.2 |
| | After 3 months storage at 40°C | 6.3 | 6.4 | 6.3 | 6.2 |
| Property/Coloration | immediately after preparation | No coloring | No coloring | No coloring | No coloring |
| | After 1 month storage at 40°C | No coloring | No coloring | No coloring | No coloring |
| | After 3 months storage at 40°C | No coloring | No coloring | No coloring | No coloring |
| Property/Shape retention | immediately after preparation | ○ | ○ | ○ | ○ |
| | After 1 month storage at 40°C | ○ | ○ | ○ | ○ |
| | After 3 months storage at 40°C | ○ | ○ | ○ | ○ |
| Feeling of use/Freshness | immediately after preparation | ○ | ○ | ○ | ○ |
| | After 1 month storage at 40°C | ○ | ○ | ○ | ○ |
| | After 3 months storage at 40°C | ○ | ○ | ○ | ○ |
| Feeling of use/ Spreadability on the skin | immediately after preparation | ○ | ○ | ○ | ○ |
| | After 1 month storage at 40°C | ○ | ○ | ○ | ○ |
| | After 3 months storage at 40°C | ○ | ○ | ○ | ○ |
| Feeling of use/ Scoopability with fingers | immediately after preparation | ○ | ○ | ○ | ○ |
| | After 1 month storage at 40°C | ○ | ○ | ○ | ○ |
| | After 3 months storage at 40°C | ○ | ○ | ○ | ○ |
| Feeling of use/ Rubbing dregs | immediately after preparation | ○ | ○ | ○ | ○ |
| | After 1 month storage at 40°C | ○ | ○ | ○ | ○ |
| | After 3 months storage at 40°C | ○ | ○ | ○ | ○ |

As shown in the results of the property evaluation for the compositions 17 and 18 of Table 6, it was confirmed that, even in the presence of ascorbic acid 2-glucoside, there was no decrease in "property/viscosity" of any of the compositions by formulating PEG-240/HDI copolymer bis-decyltetradeceth-20 ether and hydroxypropyl methylcellulose stearoxy ether, from immediately after preparation to after storage at 40 °C for 1 month and 3 months. In addition to this, the "property/shape retention" was also evaluated as "∘", which means that the gel was formed and it was confirmed that it retained its shape. The shape retention was also evaluated as "∘" not only immediately after preparation, but also after storage at 40 °C for 1 month and 3 months. For the pH of the compositions 17 and 18, no change was observed in pH from immediately after preparation to after storage at 40 °C for 3 months, and it was confirmed that the property of the composition was stable without change during the above period. Further, with respect to the coloration of the compositions 17 and 18, no change was observed in color of the composition from immediately after preparation to after storage at 40 °C for 3 months, and it was confirmed that the compositions were stable during this period without any change in their properties.

On the other hand, as shown in Table 6, it was confirmed that, even in the presence of ascorbic acid 2-glucoside, the feeling of use (freshness, spreadability on the skin, scoopability with fingers, and rubbing dregs) of the compositions 17 and 18 formulated with PEG-240/HDI copolymer bis-decyltetradeceth-20 ether and hydroxypropyl methylcellulose stearoxy ether was evaluated as "∘" from immediately after preparation to 3 months after storage at 40 °C, and was well maintained. The results of the storage stability of compositions 17 and 18 described above were equivalent to those of controls 5 and 6 which does not contain ascorbic acid 2-glucoside and 10% by mass aqueous potassium hydroxide, in all evaluation items.

The results in Table 6 show that the skin external compositions containing ascorbic acid 2-glucoside, which is an ascorbic acid derivative, PEG-240/HDI copolymer bis-decyltetradeceth-20 ether, which is a nonionic synthetic water-soluble polymers, and hydroxypropyl methylcellulose stearoxy ether, which is a natural water-soluble polysaccharides are remarkably superior in terms of shape retention, feeling of use, and stability. Furthermore, even when ascorbic acid derivatives, which impart many physiological functions as skin external preparations, are contained, and even when salts necessary for neutralization thereof are added, when used in combination with a non-ionic synthetic water-soluble polymer and a natural water-soluble polysaccharide, the results showed that, in terms of shape retention, the feeling of use, and the stability are equivalent to those of a mere skin external composition that does not contain an ascorbic acid derivative.

### <Experiment 4: Preparation of gel-type skin external compositions containing an L-ascorbic acid derivative and evaluation of their properties and feeling of use>

The evaluation of its properties and feeling of use of gel-type skin external compositions containing an L-ascorbic acid derivative was performed by varying the composition ratio of L-ascorbic acid derivative, nonionic synthetic water-soluble polymer, and natural water-soluble polysaccharide.

### <Experiment 4-1 : Preparation of a gel-type skin external composition containing an L-ascorbic acid derivative>

Compositions 19 and 20 were prepared in the same manner as in Experiment 1-1, except that ascorbic acid 2-glucoside was used as an ascorbic acid derivative, PEG-240/HDI copolymer bis-decyltetradeceth-20 ether was used as a nonionic synthetic water-soluble polymer, and cellulose derivative was used as a natural water-soluble polysaccharide, respectively, and the formulating composition was changed. The formulating composition is shown in Table 7, and in detail, for composition 19, the amount of ascorbic acid 2-glucoside was 5% by mass, the amount of PEG-240/HDI copolymer bis-decyltetradeceth-20 ether was 0.5% by mass, and the amount of hydroxypropyl methylcellulose stearoxy ether was 0.25% by mass. For composition 20, the amount of (ascorbic acid 2-glucoside was 0.01% by mass, the amount of PEG-240/ decyltetradecess-20/HDI) copolymer was 1.0% by mass, and the amount of hydroxypropyl methylcellulose stearoxy ether was 0.5% by mass.

<Experiment 4-2: Evaluation of the properties and feeling of use of gel-type skin external composition containing an L-ascorbic acid derivative>

The properties and the feeling of use of compositions 19 and 20 prepared in Experiment 4-1 were evaluated in the same manner as in Experiment 2-2, and the results are shown in Table 8. The results for composition 17 shown in Table 8 were transcribed from Table 6. For comparison, "mass ratio" was added to the bottom line of Table 8.

**[Table 7]**

| | Composition 17 | Composition 19 | Composition 20 |
|---|---|---|---|
| Ascorbic acid 2-glucoside | 2 | 5 | 0.01 |
| PEG-240/HDI copolymer bis-decyltetradeceth-20 ether | 1.2^{*} | 0.5^{*} | 1.0^{*} |
| Hydroxypropyl methylcellulose stearoxy ether | 0.2 | 0.25 | 0.5 |
| Pentylene glycol | 1.5 | 1.5 | 1.5 |
| Citrate buffer | 2 | 2 | 2 |
| 10% by mass of potassium hydroxide aqueous solution | q.s. | q.s. | q.s. |
| Deionized water | remaining | remaining | remaining |
| Total | 100 | 100 | 100 |

| | | | |
|---|---|---|---|
| (unit: % by mass) ^{*} : As dry solid content of PEG-240/HDI copolymer bis-decyltetradeceth-20 | | | |

**[Table 8]**

| | Composition 17 | Composition 19 | Composition 20 |
|---|---|---|---|
| Property/Viscosity (mPa·s) | 6665 | 4821 | 10696 |
| Property/Shape retention | ○ | ○ | ○ |
| Feeling of use/Freshness | ○ | ○ | ○ |
| Feeling of use/Spreadability on the skin | ○ | ○ | ○ |
| Feeling of use/Scoopability with fingers | ○ | ○ | ○ |
| Feeling of use/Rubbing dregs | ○ | ○ | ○ |
| Mass ratio of Ascorbic acid 2-glucoside : PEG-240/HDI copolymer bis-decyltetradeceth-20 ether: Hydroxypropyl methylcellulose stearoxy ether | 1 : 0.6 : 0.1 | 1 : 0.1 : 0.05 | 1 : 100 : 50 |

As shown in the test results of Table 8, with respect to properties, although the "property/viscosity" of composition 19 in which the amount of ascorbic acid 2-glucoside is 5% by mass, the amount of PEG-240/HDI copolymer bis-decyltetradeceth-20 ether is 0.5% by mass, and the amount of hydroxypropyl methylcellulose stearoxy ether is 0.25% by mass (as a mass ratio, 1 : 0.1 : 0.05) was a small value and the "property/viscosity" of composition 20 in which the amount of ascorbic acid 2-glucoside is 0.01% by mass, the amount of PEG-240/HDI copolymer bis-decyltetradeceth-20 ether is 1.0% by mass, and the amount of hydroxypropyl methylcellulose stearoxy ether is 0.5% by mass (as a mass ratio, 1 : 100 : 50) was a large value as compared with the result of composition 17 in which the amount of ascorbic acid 2-glucoside is 2% by mass, the amount of PEG-240/HDI copolymer bis-decyltetradeceth-20 ether is 1.2% by mass, and the amount of hydroxypropyl methylcellulose stearoxy ether is 0.2% by mass (as a mass ratio, 1 : 0.6 : 0.1) (also shown in Table 8), the "property/shape retention" of compositions 19 and 20 were both evaluated as "∘", that is, the gel was formed and retained its shape. Furthermore, its feeling of use was evaluated as "∘" in all of "freshness", "spreadability on the skin", "scoopability with fingers", and "rubbing dregs". These results were similar to those of composition 17 prepared in Experiment 3. The above results indicate that the mass ratio of ascorbic acid 2-glucoside, PEG-240/HDI copolymer bis-decyltetradeceth-20 ether, and hydroxypropyl methylcellulose stearoxy ether is preferably in the range of 1 : 0.1 to 100 : 0.05 to 50, respectively.

The results of Table 8 show that, in the skin external composition containing ascorbic acid 2-glucoside, when ascorbic acid 2-glucoside, nonionic synthetic water-soluble polymer, and natural water-soluble polysaccharide are formulated in a mass ratio on a dry solid basis thereof in a range of 1 : 0.1 to 100 : 0.05 to 50, the shape retention and the feeling of use thereof are excellent.

Hereinafter, the present invention will be specifically described based on Examples, but the present invention is not limited thereto.

### EXAMPLE 1

### <Gel-type skin external composition for whitening>

To 85 parts by mass of deionized water, 2 parts by mass of ascorbic acid 2-glucoside (product name "AA2G (registered trademark)", sold by Hayashibara Co., Ltd., Okayama, Japan) as an ascorbic acid derivative, 4 parts by mass of PEG-240/HDI copolymer bis-decyltetradeceth-20 ether (product name "Adekanol GT-730", sold by ADEKA Corporation, Tokyo, Japan) as a nonionic synthetic water-soluble polymer, 0.2 parts by mass of hydroxypropyl methylcellulose stearoxy ether (product name "Sangelose 60L", sold by Daido Chemical Corporation, Osaka, Japan) as a natural water-soluble polysaccharide, 2 parts by mass of citric acid buffer, 2.5 parts by mass of pentylene glycol (product name "HYDROLITE-5", sold by Symrise K.K., Tokyo, Japan), and an appropriate amount of 10% by mass sodium hydroxide solution were uniformly mixed to obtain a gel-type skin external composition for whitening. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for whitening was extremely stable.

### EXAMPLE 2

### <Gel-type skin external composition for lightening>

To 70 parts by mass of deionized water, 10 parts by mass of ascorbic acid 2-glucoside sodium salt (manufactured by Hayashibara Co., Ltd., Okayama, Japan) as an ascorbic acid derivative, 1 part by mass of PEG-240/HDI copolymer bis-decyltetradeceth-20 ether (product name "Adekanol GT-700", sold by ADEKA Corporation, Tokyo, Japan) as a nonionic synthetic water-soluble polymer, 1 part by mass of hydroxypropyl methylcellulose stearoxy ether (product name "Sangelose 90L", sold by Daido Chemical Corporation, Osaka, Japan) as a natural water-soluble polysaccharide, 10 parts by mass of 1,3-butylene glycol (product name "1,3-butylene glycol", sold by Daicel Corporation, Osaka, Japan), 2 parts by mass of citric acid buffer, 0.8 parts by mass of phenoxyethanol (product name "Hisolve EPH", sold by TOHO Chemical Industry Co., Ltd., Tokyo, Japan) were uniformly mixed with an appropriate amount of 10% by mass potassium hydroxide solution to obtain a gel-type skin external composition for lightening. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for lightening was extremely stable.

### EXAMPLE 3

### <Gel-type skin external composition for brightening>

To 60 parts by mass of deionized water, 5 parts by mass of ascorbic acid 2-glucoside (product name "AA2G (registered trademark)", sold by Hayashibara Co., Ltd., Okayama, Japan) and 5 parts by mass of ascorbic acid 2-glucoside potassium salt (manufactured by Hayashibara Co., Ltd., Okayama, Japan) as ascorbic acid derivatives, 4 parts by mass of PEG-240/HDI copolymer bis-decyltetradeceth-20 ether (product name "Adekanol GT-730", sold by ADEKA Corporation, Tokyo, Japan) as a nonionic synthetic water-soluble polymer, 1 part by mass of xanthan gum (product name "Echo Gum/Keltrol T", sold by DSP GOKYO Food & Chemical Co., Ltd., Osaka, Japan) as a natural water-soluble polysaccharide, 10 parts by mass of glycerin (product name "Concentrated Glycerin for Cosmetic Products", Kao Corporation, Tokyo, Japan) , 10 parts by mass of 1,3-butylene glycol (product name "1,3-butylene glycol", sold by Daicel Corporation, Osaka, Japan), 2 parts by mass of citric acid buffer solution, 2.5 parts by mass of pentylene glycol (product name "HYDROLITE-5", sold by Symrise K.K., Tokyo, Japan), and an appropriate amount of 10% by mass potassium hydroxide solution were uniformly mixed to obtain a gel-type skin external composition for brightening. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for brightening was extremely stable.

### EXAMPLE 4

### <Gel-type skin external composition for moisturizing>

To 89.4 parts by mass of deionized water, 0.01 parts by mass of ascorbic acid 2-glucoside (product name "Asco Fresh (registered trademark)" sold by Hayashibara Co., Ltd., Okayama, Japan) as an ascorbic acid derivative, 1 part by mass of PEG-240/HDI copolymer bis-decyltetradeceth-20 ether (product name "Adekanol GT-700", sold by ADEKA Corporation, Tokyo, Japan) as a nonionic synthetic water-soluble polymer, 0.1 parts by mass of tamarind gum (product name "Glyloid 6C", sold by DSP GOKYO Food & Chemical Co., Ltd., Osaka, Japan) as a natural water-soluble polysaccharide, 5 parts by mass of trehalose (product name "Trehalose (cosmetics grade)", sold by Hayashibara Co., Ltd., Okayama, Japan), 2 parts by mass of citric acid buffer, 2.5 parts by mass of pentylene glycol (product name "HYDROLITE-5", sold by Symrise K.K., Tokyo, Japan) were uniformly mixed to obtain a gel-type skin external composition for moisturizing. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for moisturizing was extremely stable.

### EXAMPLE 5

### <Gel-type skin external composition for moisturizing>

To 90.9 parts by mass of deionized water, 0.1 parts by mass of sodium ascorbate (product name "Sodium Ascorbate", sold by BASF Japan Ltd., Tokyo, Japan) as an ascorbic acid derivative, 4 parts by mass of polyvinyl alcohol (product name "Gohsenol EG-05C", sold by Mitsubishi Chemical Corporation, Tokyo, Japan) as a nonionic synthetic water-soluble polymer, 0.1 parts by mass of hydroxyethyl cellulose (product name "HEC CF-G", sold by Sumitomo Seika Chemicals Co., Ltd., Osaka, Japan) and 0.1 parts by mass of pullulan (product name "Pullulan", sold by Hayashibara Co., Ltd., Okayama, Japan) as natural water-soluble polysaccharides, 2 parts by mass of citric acid buffer, and 2.8 parts by mass of pentylene glycol (product name "HYDROLITE-5", sold by Symrise K.K., Tokyo, Japan) were uniformly mixed to obtain a gel-type skin external composition for moisturizing. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for moisturizing was extremely stable.

### EXAMPLE 6

### <Gel-type serum>

To 60 parts by mass of deionized water, 2 parts by mass of ascorbic acid 2-glucoside (product name "AA2G (registered trademark)," sold by Hayashibara Co., Ltd., Okayama, Japan) as an ascorbic acid derivative, 2 parts by mass of polyvinyl alcohol (product name "Gohsenol EG-05C", sold by Mitsubishi Chemical Corporation, Tokyo, Japan) as a nonionic synthetic water-soluble polymer, 0.3 parts by mass of sodium alginate (product name "Algecolloid", sold by Yamakawa & Co., Ltd., Tokyo, Japan) as a natural water-soluble polysaccharide, 8 parts by mass of sorbitol (product name "Sorbitol Kao", sold by Kao Corporation, Tokyo, Japan), 5 parts by mass of1,3-butylene glycol (product name "1,3-butylene glycol",sold by Daicel Corporation, Osaka, Japan), PEG-6,PEG-32 (product name "PEG#1500", sold by NOF Corporation, Tokyo, Japan), 7 parts by mass of PEG-10 laurate sorbitan (product name "Nonion LT-210", sold by NOF Corporation, Tokyo, Japan), 0.2 parts by mass of olive oil, 7 parts by mass of citric acid buffer, 2.5 parts by mass of pentylene glycol (product name "HYDROLITE-5", sold by Symrise K.K., Tokyo, Japan), and an appropriate amount of 10% by mass potassium hydroxide solution were uniformly mixed to obtain a gel-type serum. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type serum was extremely stable.

### EXAMPLE 7

To 54.4 parts by mass of deionized water, 2 parts by mass of ascorbic acid 2-glucoside (product name "AA2G (registered trademark)," sold by Hayashibara Co., Ltd., Okayama, Japan) as an ascorbic acid derivative, 1 part by mass of ethylene oxide propylene oxide block copolymer (product name "Creagel Crystal AF", sold by Shima Trading Co., Ltd., Tokyo, Japan) and 1 part by mass of polyurethane (product name "Adekanol GT-930", sold by ADEKA Corporation, Tokyo, Japan) as nonionic synthetic water-soluble polymers, 0.1 parts by mass of pullulan (product name "Pullulan", sold by Hayashibara Co., Ltd., Okayama, Japan) as a natural water-soluble polysaccharide, and 7 parts by mass of polyglyceryl isostearate-2 (product name "NIKKOL DGMIS", sold by Nikko Chemicals Co., Ltd., Tokyo, Japan), 5 parts by mass of Sorbes-30 Tetraoleate (product name "NIKKOL GO-430NV", sold by Nikko Chemicals Co., Ltd., Tokyo, Japan), 4 parts by mass of Glycerin (product name "Concentrated Glycerin" sold by Kao Corporation, Tokyo, Japan), 6 parts by mass of dipropylene glycol (product name "DPG", sold by Dow Chemical Japan Co., Ltd., Tokyo, Japan) 5 parts by mass of dimethicone (product name "SH200C", sold by Dow Chemical Japan Co., Ltd., Tokyo, Japan), 5 parts by mass of cyclopentasiloxane (product name "KF-995", sold by Shin-Etsu Chemical Co., Ltd., Tokyo, Japan), 5 parts by mass of liquid paraffin (product name "Moresco White P-70", sold by MORESCO Corporation, Hyogo, Japan), 2 parts by mass of citric acid buffer, 2.5 parts by mass of pentylene glycol (product name "HYDROLITE-5", sold by Symrise K.K., Tokyo, Japan), and an appropriate amount of 10% by mass potassium hydroxide solution were uniformly mixed to obtain a gel-type serum. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type serum was extremely stable.

### EXAMPLE 8

### <Gel-type skin external composition for cooling sensation>

To 65 parts by mass of deionized water, 2 parts by mass of ascorbic acid 2-glucoside (product name "AA2G (registered trademark)," sold by Hayashibara Co., Ltd., Okayama, Japan) as an ascorbic acid derivative, 2 parts by mass of ethylene oxide propylene oxide block copolymer (product name "Creagel Crystal AF", sold by Shima Trading Co., Ltd., Tokyo, Japan) and 2 parts by mass of PEG-240/HDI copolymer bis-decyltetradeceth-20 ether (product name "Adekanol GT-730", sold by ADEKA Corporation, Tokyo, Japan) as nonionic synthetic water-soluble polymers, 0.2 parts by mass of guar gum (product name "SUPERGEL CSA200/50", sold by Sansho Co., Ltd., Osaka, Japan) as a natural water-soluble polysaccharide, 10 parts by mass of glycerin (product name "Concentrated Glycerin for Cosmetic Products", sold by Kao Corporation, Tokyo, Japan), 10 parts by mass of ethanol, 0.1 parts by mass of menthol (product name "L-menthol", sold by The Suzuki Menthol Co., Ltd., Hyogo, Japan), 2 parts by mass of citric acid buffer, 0.8 parts by mass of phenoxyethanol (product name "Hisolve EPH", sold by TOHO Chemical Industry Co., Ltd., Tokyo, Japan), and an appropriate amount of 10% by mass potassium hydroxide solution were uniformly mixed to obtain a gel-type skin external composition for cooling sensation. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for cooling sensation was extremely stable.

### EXAMPLE 9

### <Gel-type skin external composition for warm sensation>

To 84.9 parts by mass of deionized water, 0.1 parts by mass of ascorbic acid 2-glucoside (product name "AA2G (registered trademark)", sold by Hayashibara Co., Ltd., Okayama, Japan) as an ascorbic acid derivative, 1 part by mass of ethylene oxide propylene oxide block copolymer (product name "Creagel Crystal AF", sold by Shima Trading Co., Ltd., Osaka, Japan) and 0.5 parts by mass of PEG-240/HDI copolymer bis-decyltetradeceth-20 ether (product name "Adekanol GT-700", sold by ADEKA Corporation, Tokyo, Japan) as nonionic synthetic water-soluble polymers, and 0.2 parts by mass of xanthan gum (product name "KELTROL CG-BT", sold by Sumitomo Seika Chemicals Co., Ltd., Osaka, Japan) as a natural water-soluble polysaccharide, 10 parts by mass of 1,3-butylene glycol (product name "1,3-butylene glycol", sold by Daicel Corporation, Osaka, Japan), 0.5 parts by mass of glycosyl hesperidin (product name "Alpha Glucosyl Hesperidin", sold by Hayashibara Co., Ltd., Japan), 2 parts by mass of citric acid buffer, and 0.8 parts by mass of phenoxyethanol (product name "Hisolve EPH", Sold by TOHO Chemical Industry Co., Ltd., Tokyo, Japan) were uniformly mixed to obtain a gel-type skin external composition for warm sensation. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for warm sensation was extremely stable.

### EXAMPLE 10

### <Gel-type skin external composition for massage>

To 70.8 parts by mass of deionized water, 2 parts by mass of trehalose (product name "TREHA", sold by Hayashibara Co., Ltd., Okayama, Japan), 0.1 parts by mass of ascorbic acid 2-glucoside (product name "AA2G (registered trademark)", sold by Hayashibara Co., Ltd., Okayama, Japan) as an ascorbic acid derivative, 4 parts by mass of polyvinyl alcohol (product name "Gohsenol EG-05C", sold by Mitsubishi Chemical Corporation, Tokyo, Japan) as a nonionic synthetic water-soluble polymer, 0.3 parts by mass of hydroxyethyl cellulose (product name "HEC CF-W", sold by Sumitomo Seika Chemicals Co., Ltd., Osaka, Japan) as a natural water-soluble polysaccharide, and 4 parts by mass of hyaluronic acid (product name "HYALURONSAN HA-LQ", sold by Kewpie Corporation, Tokyo, Japan), 10 parts by mass of 1,3-butylene glycol (product name "1,3-butylene glycol", sold by Daicel Corporation, Osaka, Japan), 10 parts by mass of glycerin (product name "Concentrated Glycerin for Cosmetic Products", sold by Kao Corporation, Tokyo, Japan), 2 parts by mass of citric acid buffer, 0.8 parts by mass of phenoxyethanol (product name "Hisolve EPH", Sold by TOHO Chemical Industry Co., Ltd., Tokyo, Japan) were uniformly mixed to obtain a gel-type skin external composition for massage. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type skin external composition for massage was extremely stable.

### EXAMPLE 11

### <Gel-type cleaning agent>

To 76.8 parts by mass of deionized water, 0.1 parts by mass of ascorbic acid 2-glucoside (product name "AA2G (registered trademark)," sold by Hayashibara Co., Ltd., Okayama, Japan) as an ascorbic acid derivative, 1 part by mass of ethylene oxide propylene oxide block copolymer (product name "Creagel Crystal AF", sold by Shima Trading Co., Ltd., Tokyo, Japan) as a nonionic synthetic water-soluble polymer, 0.3 parts by mass of hydroxyethyl cellulose (product name "HEC CF-X", sold by Sumitomo Seika Chemicals Co., Ltd., Osaka, Japan) as a natural water-soluble polysaccharide, 6 parts by mass of dipropylene glycol (product name "DPG", sold by Dow Chemical Japan Co., Ltd., Tokyo, Japan), 6 parts by mass of 1,3-butylene glycol (product name "1,3-butylene glycol", sold by Daicel Corporation, Osaka, Japan), PEG-75 (product name "PEG400", sold by NOF Corporation, Tokyo, Japan), 1 part by mass of POE (20) cocoyl methyl taurine sodium salt (product name "Neoscope CN-30", sold by TOHO Chemical Industry Co., Ltd., Tokyo, Japan), 2 parts by mass of citric acid buffer, and 0.8 parts by mass of phenoxyethanol (product name "High Solve EPH", sold by TOHO Chemical Industry Co., Ltd., Tokyo, Japan) were uniformly mixed to obtain a gel-type cleaning agent. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type cleaning agent was extremely stable.

### EXAMPLE 12

### <Gel-type UV inhibitor>

To 69.2 parts by mass of deionized water, 1 part by mass of magnesium ascorbyl phosphate (product name "L-Ascorbic Acid Phosphate Magnesium Salt", sold by FUJIFILM Wako Pure Chemical Corporation, Osaka, Japan) as an ascorbic acid derivative, 3 parts by mass of polyurethane (product name "Adekanol GT-930", sold by ADEKA Corporation, Tokyo, Japan) as a nonionic synthetic water-soluble polymer, 1 part by mass of soluble dextrin (product name "Fibryxa (registered trademark)", sold by Hayashibara Co., Ltd., Okayama, Japan) and 10 parts by mass of cyclodextrin (product name "CD102", sold by Roquette Japan K.K., Tokyo, Japan) as natural water-soluble polysaccharides, 5 parts by mass of 1,3-butylene glycol (product name "1,3-butylene glycol", sold by Daicel Corporation, Osaka, Japan), 3 parts by mass of polyglyceryl stearate-10 (product name "NIKKOL Decaglyn 1-SV", sold by Nikko Chemicals Co., Ltd., Tokyo, Japan), 5 parts by mass of titanium Oxide (product name "Titanium Oxide", sold by Ishihara Sangyo Kaisha, Ltd., Osaka, Japan), 2 parts by mass of citric acid buffer, and 0.8 parts by mass of phenoxyethanol (product name "Hisolve EPH", sold by TOHO Chemical Industry Co., Ltd., Tokyo, Japan) were uniformly mixed to obtain a gel-type UV inhibitor. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type UV inhibitor was extremely stable.

### EXAMPLE 13

### <Gel-type wash-off pack>

To 81.1 parts by mass of deionized acid, 0.1 parts by mass of ascorbyl sodium phosphate (product name "L-ascorbyl sodium phosphate" sold by BASF Japan Ltd., Tokyo, Japan) as an ascorbic acid derivative, 5 parts by mass of polyvinyl alcohol (product name "Gohsenol EG-05C" sold by Mitsubishi Chemical Corporation, Tokyo, Japan) as a non-ionic synthetic water-soluble polymer, 1 part by mass of locust bean gum (product name "PHYTALURONATE PF " sold by DSM Nutrition Japan Co., Ltd., Tokyo, Japan) as a natural water-soluble polysaccharide, 10 parts by mass of sorbitol (product name "Sorbitol Kao" sold by Kao Corporation, Tokyo, Japan), 2 parts by mass of citric acid buffer solution, and 0.8 parts by mass of phenoxyethanol (product name "Hisolve EPH", sold by TOHO Chemical Industry Co., Ltd., Tokyo, Japan) were uniformly mixed to obtain a gel-type wash-off pack. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and even after storage for 3 months, the shape retention property, the feeling of use were maintained, and the gel-type wash-off pack was extremely stable.

### EXAMPLE 14

### <Gel-type skin external composition for whitening>

To 79.3 parts by mass of deionized water, 2 parts by mass of ascorbic acid 2-glucoside (product name "AA2G (registered trademark)", sold by Hayashibara Co., Ltd., Okayama, Japan) as an ascorbic acid derivative, 4 parts by mass of PEG-240/HDI copolymer bis-decyltetradeceth-20 ether (product name "Adekanol GT-730", sold by ADEKA Corporation, Tokyo, Japan) as a nonionic synthetic water-soluble polymer, 0.2 parts by mass of hydroxypropyl methylcellulose stearoxy ether (product name "Sangelose 60L", sold by Daido Chemical Corporation, Osaka, Japan) as a natural water-soluble polysaccharide, 10 parts by mass of diethoxyethyl succinate (product name "Crodamol DES-LQ-(JP)", sold by Croda Japan K.K., Tokyo, Japan), 2 parts by mass of citric acid buffer, 2.5 parts by mass of pentylene glycol (product name "HYDROLITE-5", sold by Symrise K.K., Tokyo, Japan), and an appropriate amount of 10% by mass sodium hydroxide solution were uniformly mixed to obtain a gel-type skin external composition for whitening. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and also that the transparency upon application was good, and even after storage for 3 months, the shape retention property, the feeling of use, and the transparency upon application were maintained, and the gel-type skin external composition for whitening was extremely stable.

### EXAMPLE 15

### <Gel-type skin external composition for lightening >

To 74.3 parts by mass of deionized water, 2 parts by mass of ascorbic acid 2-glucoside (product name "AA2G (registered trademark)", sold by Hayashibara Co., Ltd., Okayama, Japan) as an ascorbic acid derivative, 4 parts by mass of PEG-240/HDI copolymer bis-decyltetradeceth-20 ether (product name "Adekanol GT-730", sold by ADEKA Corporation, Tokyo, Japan) as a nonionic synthetic water-soluble polymer, 0.2 parts by mass of hydroxypropyl methylcellulose stearoxy ether (product name "Sangelose 60L", sold by Daido Chemical Corporation, Osaka, Japan) as a natural water-soluble polysaccharide, 5 parts by mass of diethoxyethyl adipate (FUJIFILM Wako Pure Chemical Corporation, Osaka, Japan), 10 parts by mass of ethanol, 2 parts by mass of citric acid buffer solution, 2.5 parts by mass of pentylene glycol (product name "HYDROLITE-5", sold by Symrise K.K., Tokyo, Japan), and an appropriate amount of 10% by mass sodium hydroxide solution were uniformly mixed to obtain a gel-type skin external composition for lightening. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and also that the transparency upon application was good, and even after storage for 3 months, the shape retention property, the feeling of use, and the transparency upon application were maintained, and the gel-type skin external composition for lightening was extremely stable.

### EXAMPLE 16

### <Gel-type skin external composition for brightening >

To 76.8 parts by mass of deionized water, 2 parts by mass of ascorbic acid 2-glucoside (product name "AA2G (registered trademark)", sold by Hayashibara Co., Ltd., Okayama, Japan) as an ascorbic acid derivative, 4 parts by mass of PEG-240/HDI copolymer bis-decyltetradeceth-20 ether (product name "Adecanol GT-730", sold by ADEKA Corporation, Tokyo, Japan) as a nonionic synthetic water-soluble polymer, 0.2 parts by mass of hydroxypropyl methylcellulose stearoxy ether (product name "Sangelose 60L", sold by Daido Chemical Corporation, Osaka, Japan) as a natural water-soluble polysaccharide, 2.5 parts by mass of diethoxyehyl sebacate (sold by Kanto Chemical Co., Inc., Tokyo, Japan), 10 parts by mass of ethanol, 2 parts by mass of citric acid buffer solution, 2.5 parts by mass of pentylene glycol (product name "HYDROLITE-5", sold by Symrise K.K., Tokyo, Japan), and an appropriate amount of 10% by mass sodium hydroxide solution were uniformly mixed to obtain a gel-type skin external composition for brightening. When the properties of the product immediately after preparation and after storage at 40 °C for 3 months were tested in the same manner as in Experiment 3, the results were obtained that the shape retention property and the feeling of use (freshness, spreadability on the skin, scoopability with fingers, rubbing dregs) were good, and also that the transparency upon application was good, and even after storage for 3 months, the shape retention property, the feeling of use, and the transparency upon application were maintained, and the gel-type skin external composition for brightening was extremely stable.

## Claims

1. A gel-type skin external composition comprising a L-ascorbic acid derivative, a nonionic synthetic water-soluble polymer, and a natural water-soluble polysaccharide.

2. The gel-type skin external composition of claim 1, wherein the L-ascorbic acid derivative is L-ascorbic acid 2-glucoside.

3. The gel-type skin external composition of claim 1 or 2, wherein the nonionic synthetic water-soluble polymer is one or more members selected from PEG-240/HDI copolymer bis-decyltetradeceth-20 ether, polyurethane, polyvinyl alcohol, ethylene oxide-propylene oxide block copolymer, and derivatives thereof.

4. The gel-type skin external composition of any one of claims 1 to 3, wherein the natural water-soluble polysaccharide is one or more members selected from soluble cellulose, xanthan gum, guar gum, tamarind gum, locust bean gum, soluble starch, soluble dextrin, cyclodextrin, pullulan and derivatives thereof.

5. The gel-type skin external composition of any one of claims 1 to 4, wherein the composition is excellent in shape retention, feeling of use, and stability.

6. The gel-type skin external composition of any one of claims 1 to 5, wherein a mass ratio of the L-ascorbic acid derivative, the nonionic synthetic water-soluble polymer, and the natural water-soluble polysaccharide on a dry solid basis is in the range of 1 : 0.01 - 500 : 0.01 - 500.

7. The gel-type skin external composition of any one of claims 1 to 6, further comprising a polyoxyethylene dicarboxylic acid ester.

8. The gel-type skin external composition of claim 7, wherein the polyoxyethylene dicarboxylic acid ester is one or more members selected from diethoxyethyl succinate, diethoxyethyl adipate, and diethoxyethyl sebacate.
